# EUROPÄISCHE PATENTSCHRIFT

(11) **EP 1 332 221 B1**
(45) Veröffentlichungstag und Bekanntmachung des Hinweises auf die Patenterteilung: **28.06.2006**
(21) Anmeldenummer: 01993696.2
(22) Anmeldetag: 07.11.2001
(51) Int. Cl.: C12P 1/00, C12N 9/00, C12P 19/04, C12P 19/12

(54) **ENZYMKATALYSE IN GEGENWART IONISCHER FLÜSSIGKEITEN**
ENZYME CATALYSIS IN THE PRESENCE OF IONIC LIQUIDS
CATALYSE ENZYMATIQUE EN PRESENCE DE LIQUIDES IONIQUES

(30) Priorität: 08.11.2000 EP 00124195
(43) Veröffentlichungstag der Anmeldung: 06.08.2003
(73) Patentinhaber: Solvent Innovation GmbH, 50829 Köln (DE)
(72) Erfinder: KRAGL, Udo, 18198 Kritzmow (DE); KAFTZIK, Nicole, 18055 Rostock (DE); SCHÖFER, Sonja, 40789 Monheim (DE); WASSERSCHEID, Peter, 50829 Koeln (DE)
(74) Vertreter: Weber, Thomas
(86) Internationale Anmeldenummer: PCT/EP2001/012869
(87) Internationale Veröffentlichungsnummer: WO 2002/038784

(56) Entgegenhaltungen:
- EP-A1- 0 226 563
- DATABASE CHEMABS [Online] CHEMICAL ABSTRACTS SERVICE, COLUMBUS, OHIO, US; Database accession no. 1999:144145, ALSTON, WILLIAM C., II ET AL: "Enzymic reactions in ionic liquids" retrieved from STN XP002163388 & BOOK OF ABSTRACTS, 217TH ACS NATIONAL MEETING, ANAHEIM, CALIF., MARCH 21-25 (1999), BIOT-131 PUBLISHER: AMERICAN CHEMICAL SOCIETY, WASHINGTON, D. C.,
- DATABASE CHEMABS [Online] CHEMICAL ABSTRACTS SERVICE, COLUMBUS, OHIO, US; CULL, S. G. ET AL: "Room-temperature ionic liquids as replacements for organic solvents in multiphase bioprocess operations" retrieved from STN Database accession no. 133:163060 XP002163389 & BIOTECHNOL. BIOENG. (2000), 69(2), 227-233,
- DATABASE CHEMABS [Online] CHEMICAL ABSTRACTS SERVICE, COLUMBUS, OHIO, US; ERBELDINGER, MARKUS ET AL: "Enzymatic catalysis of formation of Z-aspartame in ionic liquid - an alternative to enzymatic catalysis in organic solvents" retrieved from STN Database accession no. 133:321046 XP002163390 & BIOTECHNOL. PROG. (2000), 16(6), 1131-1133,
- DATABASE CHEMABS [Online] CHEMICAL ABSTRACTS SERVICE, COLUMBUS, OHIO, US; LAU, R. MADEIRA ET AL: "Lipase-Catalyzed Reactions in Ionic Liquids" retrieved from STN Database accession no. 134:147417 XP002163391 & ORG. LETT. (2000), 2(26), 4189-4191,
- DATABASE BIOSIS [Online] BIOSCIENCES INFORMATION SERVICE, PHILADELPHIA, PA, US; November 2000 (2000-11) ERBELDINGER MARKUS ET AL: "Enzymatic catalysis of formation of Z-aspartame in ionic liquid: An alternative to enzymatic catalysis in organic solvents." Database accession no. PREV200100074842 XP002163392 & BIOTECHNOLOGY PROGRESS, Bd. 16, Nr. 6, November 2000 (2000-11), Seiten 1129-1131, ISSN: 8756-7938
- PATENT ABSTRACTS OF JAPAN vol. 2000, no. 12, 3. Januar 2001 (2001-01-03) & JP 2000 245496 A (MEIJI MILK PROD CO LTD), 12. September 2000 (2000-09-12)
- DATABASE WPI Section Ch, Week 199249 Derwent Publications Ltd., London, GB; Class D16, AN 1992-405174 XP002189948 & KR 9 108 732 B (KOREA ADV INST SCI & TECHN), 19. Oktober 1991 (1991-10-19)

## Beschreibung

Die Erfindung betrifft Zusammensetzungen umfassend ein Enzym sowie ionische Flüssigkeiten sowie ein Verfahren zur Durchführung Enzymkatalysierter Reaktionen in Gegenwart von ionischen Flüssigkeiten.

Enzyme haben als Biokatalysatoren inzwischen einen festen Platz für Umsetzungen im Labor- und Industriemaßstab eingenommen. Dennoch treten trotz aller Erfolge bei enzymatischen Reaktionen immer noch Probleme auf wie beispielsweise
- niedrige Produktivitäten aufgrund zu geringer Eduktlöslichkeiten;
- niedrige Ausbeuten bei Gleichgewichtsreaktionen;
- unzureichende Selektivität bei regio- oder stereoselektiven Umsetzungen;
- Produktinhibierung;
- Auftreten von Nebenreaktionen (Parallel-, Folgereaktionen).

Bekannt sind Ansätze, diese Probleme durch Zusatz organischer Lösungsmittel (G. Carrea, S. Riva, Angew. Chem. 2000, 112, 2312; J. M. S. Cabral, M. R. Aires-Barros, H. Pinheiro, D. M. F. Prazeres, J. Biotechnol. 1997, 59, 133; M. N. Gupta, Eur. J. Biochem. 1992, 203, 25), Zusatz von Salzen (A. M. Blinkorsky, Y. L. Khmelnitzky, J. S. Dordick, J. Am. Chem. Soc. -1999, 116, 2697) oder durch Durchführung der Reaktion in Mikroemulsionen (B. Orlich, R. Schomäcker 1999, 65, 357-362) zu lösen. Oft sind die dadurch erzielten Verbesserungen jedoch nicht signifikant und rechtfertigen nicht den zusätzlichen Aufwand, oder die Enzymstabilität nimmt unter diesen Bedingungen stark ab (G. Carrea, S. Riva, Angew. Chem. 2000, 112, 2312).

Ionische Flüssigkeiten sind bei niedrigen Temperaturen (<100 °C) schmelzende Salze, die eine neuartige Klasse von Lösungsmitteln mit nichtmolekularem, ionischem Charakter darstellen. Auch wenn erste Vertreter bereits seit 1914 bekannt sind, werden ionische Flüssigkeiten erst in den letzten 15 Jahren intensiv als Lösungsmittel für chemische Umsetzungen untersucht. Ionische Flüssigkeiten besitzen keinen messbaren Dampfdruck. Dies ist aus verfahrenstechnischer Hinsicht ein großer Vorteil, da auf diese Weise die destillative Trennung eines Reaktionsgemisches als effektive Methode zur Produktabtrennung möglich ist. Die bekannten Probleme durch Azeotropbildung zwischen Lösungsmitteln und Produkten treten nicht auf. Ionische Flüssigkeiten sind bis über 200 °C temperaturstabil. Durch geeignete Wahl von Kation und Anion ist eine stufenweise Einstellung der Polarität und damit eine Abstimmung der Löslichkeitseigenschaften möglich. Die Bandbreite reicht dabei von wassermischbaren ionische Flüssigkeiten über wassernichtmischbare bis hin zu solchen, die selbst mit organischen Lösungsmitteln zwei Phasen bilden. Die geschickte Ausnutzung der außerordentlichen Löslichkeitseigenschaften ist der Schlüssel zum erfolgreichen Einsatz der ionische Flüssigkeiten als neuartige Lösungsmittelklasse.

Ionische Flüssigkeiten konnten als neuartige Medien in der Zweiphasen-Katalyse oder als Medium zur flüssig-flüssig-Extraktion bereits erfolgreich eingesetzt werden (P. Wasserscheid, W. Keim, Angew. Chem. 2000, 112, 3926).

Überraschenderweise wurde erfindungsgemäß bei der Umsetzung verschiedenster Edukte mit unterschiedlichen Enzymen in Gegenwart ionischer Flüssigkeiten eine starke Erhöhung der Ausbeute und Selektivität festgestellt, die gegenüber dem bekannten Stand der Technik eine deutliche Verbesserung darstellt. Nachteilige Auswirkungen der ionischen Flüssigkeit auf die Enzymstabilität wurden nicht festgestellt, im Einzelfall wurde sogar eine stabilisierende Wirkung gefunden.

Dies ist unerwartet und überraschend, berücksichtigt man die ionische Natur der ionischen Flüssigkeiten und die damit möglichen starken Wechselwirkungen zwischen der ionischen Flüssigkeit und dem Enzym mit seinen ebenfalls geladenen Gruppen.

Ebenso wurde gefunden, dass ionische Flüssigkeiten als Co-Solventien zur Verbesserung der Löslichkeit von Edukten und Produkten eingesetzt werden können.

Alston et al offenbaren in Book of Abstracts, 217^{th} ACS National Meeting, Anaheim, Calif., March 21-25 (1999) BIOT-131, Publisher: American Chemical Society, Washington D. C. die Reaktion von Subtilisin und Chymotrypsin (jeweils Enzyme der EC.-Klasse 3.4) in 1-Butyl-3-methylimidazoliumtetrafluoroborat.

Cull et al beschreiben in Biotechno. Bioeng. (2000), 69(2), 227-233 die durch Rhodococcus R312 (EC. 3.3.2.8) katalysierte Biotransformation von 1,3-Dicyanobenzol in 1-Butyl-3-methylimidazoliumhexafluorophosphat.

Erbeldinger et al offenbaren in Biotechnol. Prog. (2000), 16(6), 1131-1133 die Synthese von Z-Aspartam in einer durch Thermolysin (EC. 3.4) katalysierten Reaktion von Carbobenzoxy-L-aspartat und L-Phenylalaninmethylesterhydrochlorid in 1-Butyl-3-methylimidazoliumhexafluorophosphat.

Lau et al offenbaren in ORG. LETT. (2000), 2(26), 4189-4191 Candida antarctica lipase (EC. 3.1.1) katalysierte Reaktionen in 1-Butyl-3-methylimidazoliumtetrafluoroborat.

Die Erfindung betrifft ein Verfahren zur Umsetzung von Substanzen (Edukten) in Gegenwart von Enzymen als Katalysator in einem Reaktionsmedium umfassend ionische Flüssigkeiten.

Die ionische Flüssigkeit kann dabei mit Wasser mischbar oder mit Wasser nicht mischbar sein. Ebenso ist eine ein-, zwei- oder mehrphasige Reaktionsführung möglich.

Bei den ionischen Flüssigkeiten handelt es sich um Verbindungen der allgemeinen Formel

[A]ₙ⁺[Y]ⁿ⁻,

wobei
n = 1 oder 2 ist und
das Anion [Y]ⁿ⁻ ausgewählt ist aus der Gruppe bestehend aus Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprat, Sulfat ([SO₄]²⁻), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12
Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-arylRest ist, der durch Halogenatome substituiert sein kann, das Kation [A]⁺ ist ausgewählt aus
- quarternären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 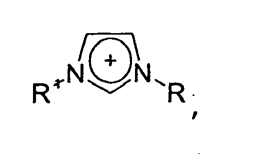
wobei der Imidazol-Kern substituiert sein "kann mit wenigstens einer Gruppe, die ausgewählt ist aus C₁-C₆-Alkyl-, C₁-C₆-Alkoxy-, C₁-C₆₋Aminoalkyl-, C₅-C₁₂-Aryl- oder C₅-C₁₂-Aryl-C₁-C₆-Alkylgruppen,
- Pyridinium-Kationen der allgemeinen Formel 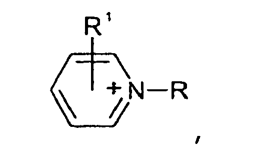
wobei der Pyridin-Kern substituiert sein kann mit mit wenigstens einer Gruppe, die ausgewählt ist aus C₁-C₆-Alkyl-, C₁-C₆-Alkoxy-, C₁₋C₆-Aminoalkyl-, C₅-C₁₂-Aryl- oder C₅-C₁₂-Aryl-C₁-C₆-Alkylgruppen,
- Pyrazolium-Kationen der allgemeinen Formel 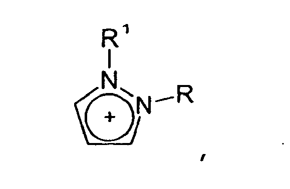
wobei der Pyrazol-Kern substituiert sein kann mit mit wenigstens einer Gruppe, die ausgewählt ist aus C₁-C₆-Alkyl-, C₁-C₆-Alkoxy-, C₁₋C₆-Aminoalkyl-, C₅-C₁₂-Aryl- oder C₅-C₁₂-Aryl-C₁-C₆-Alkylgruppen,
- und Triazolium-Kationen der allgemeinen Formel 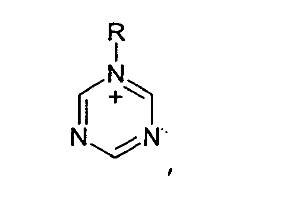
wobei der Triazol-Kern substituiert sein kann mit mit wenigstens einer Gruppe, die ausgewählt ist aus C₁-C₆-Alkyl-, C₁-C₆-Alkoxy-, C₁₋C₆-Aminoalkyl-, C₅-C₁₂-Aryl- oder C₅-C₁₂-Aryl-C₁-C₆-Alkylgruppen, und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können.

In einem weiteren Aspekt betrifft die Erfindung eine Zusammensetzung umfassend ein Enzym sowie wenigstens eine der oben definierten ionischen Flüssigkeit. Diese Zusammensetzungen können als Ausgangspunkt für die Durchführung der oben genannten enzymatisch katalysierten Reaktionen dienen. Dementsprechend können die erfindungsgemäßen Zusammensetzungen neben dem Enzym (Biokatalysator) auch die umzusetzenden Edukte (Substrate) enthalten und, bei Voranschreiten der Reaktion, selbstverständlich auch die durch die enzymatische Reaktion erhältlichen Reaktionsprodukte.

Ein noch weiterer Aspekt ist somit die Verwendung von ionischen Flüssigkeiten, insbesondere der oben definierten ionischen Flüssigkeiten, als Reaktionsmedium oder Bestandteil des Reaktionsmediums in der Biokatalyse, also der Durchführung von enzymatisch katalysierten Reaktionen an Substraten.

In einer besonderen Ausgestaltung der Erfindung können die Alkyl-, Aryl-, Arylalkyl- und Alkylaryl-Sulfonatgruppen (Anion [Y]) durch Halogenatome, insbesondere Fluor, Chlor oder Brom substituiert sein. Besonders bevorzugt sind die fluorierten, insbesondere die perfluorierten Alkyl- und obengenannten Arylsulfonate, wie das Trifluormethansulfonat (Triflat). Als nicht halogenierte Vertreter sind die Methansulfonat-, Benzolsulfonat- und die Toluolsulfonat-Gruppe zu nennen, sowie alle weiteren im Stand der Technik bekannten Sulfonat-Austrittsgruppen.

In einer weiteren Ausgestaltung der Erfindung können die Alkyl-, Aryl-, Arylalkyl- und Alkylaryl-Carboxylatgruppen durch Halogenatome, insbesondere Fluor, Chlor oder Brom substituiert sein. Besonders bevorzugt sind die fluorierten, insbesondere die perfluorierten Alkyl- und obengenannten Arylcarboxylate, wie das Trifluormethancarboxylat (Trifluoracetat; CF₃COO⁻). Als nicht halogenierte Vertreter sind die Acetat- und Benzoat-Gruppe zu nennen, sowie alle weiteren im Stand der Technik bekannten Carboxylat-Austrittsgruppen.

In bevorzugten Ausgestaltungen der Erfindung können die im Zusammenhang mit den Substituenten erwähnten C₁-C₆-Alkyl-Gruppen jeweils unabhängig voneinander durch C₂-C₄-Alkyl-Gruppen ersetzt werden. Ebenso können die im Zusammenhang mit den Substituenten erwähnten C₁-C₆-Alkoxy-Gruppen jeweils unabhängig voneinander durch C₂-C₄-Alkoxy-Gruppen ersetzt werden. In einer weiteren Alternative der Erfindung können die im Zusammenhang mit den Substituenten erwähnten C₅-C₁₂-Aryl-Gruppen jeweils unabhängig voneinander durch C₆-C₁₀-Aryl-Gruppen, die C₃-C₈-Heteroaryl-Gruppen jeweils unabhängig voneinander durch C₃-C₆-Heteroaryl-Gruppen ersetzt werden. Die Halogenatome, mit welchen die Alkyl-, Alkoxy- und Aryl-Gruppen substituiert sein können sind ausgewählt aus Fluor, Chlor, Brom und Iod, vorzugsweise Fluor, Chlor und Brom.

Ein einer bevorzugten Ausgestaltung ist der Rest R' ein linearer oder verzweigter 1 bis 8 Kohlenstoffatome enthaltender aliphatischer oder alicyclischer Alkyl- oder ein C₆-C₁₀-Aryl-, C₆-C₁₀-Aryl-C₁-C₄-alkyl- oder C₁-C₄₋Alkyl-C₆-C₁₀-Arylrest, der durch Halogenatome substituiert sein kann.

Die Kationen [A] sind beispielsweise ausgewählt aus Trimethylphenylammonium, Methyltrioctylammonium, Tetrabutylphosphonium, 3-Butyl-1-methyl-imidazolium, 3-Ethyl-1-methyl-imidazolium, N-Butylpyridinium, N-Ethylpyridinium, Diethylpyrazolium, 1-Ethyl-3-methylimidazolium, 1-Butyl-3-methylimidazolium, 1-Hexyl-3-methylimidazolium, 1-Octyl-3-methylimidazolium, 1-Decyl-3-methylimidazolium, 1-Butyl-4-methylpyridinium, 1-Butyl-3-methylpyridinium, 1-Butyl-2-methylpyridinium, 1-Butyl-pyridinium,.Butyl-Methyl-Imidazolium, Nonyl-Methyl-Imidazolium, Butyl-Methyl-Imidazolium, Hexyl-Methyl-Imdazolium, Octyl-Methyl-Imidazolium, 4-Methyl-Butyl-Pyridinium, Triethylammonium, Trieethylmethylammonium, Butylmethyl-Pyridinium, Propylammonium, Methyl-Methyl-Imidazolium, Ethyl-Methyl-Imidazolium, Butyl-Methyl-Imidazolium und Butyl-Methyl-Imidazolium.

Ionische Flüssigkeiten sowie deren Herstellung sind im Stand der Technik bekannt. Zur Synthese von ionischen Flüssigkeiten mit Hexafluorophosphat-, Tetrafluoroborat-, Bis(trifluormethylsulfonyl)amid-, Perfluoralkylsulfonat- und

Perfluoralkylcarboxylat-Ionen wird zunächst durch Reaktion eines Amins NR₁R₂R₃, eines Phosphans PR¹R²R³, eines Imidazolderivates der allgemeinen Formel R¹R²⁺N=CR³-R⁵-R³C=N⁺R¹R² oder eines Pyridiniumderivates der allgemeinen Formel R¹R²N=CR³R⁴⁺ mit einem Alkylchlorid, Alkylbromid oder Alkyliodid das entsprechende Halogenidsalz [Kation]⁺X⁻ gebildet und isoliert (F.H. Hurley, T.P. Wier, Jr., J. Electrochem. Soc. 1951, 98, 207-212; J.S. Wilkes, J.A. Levisky, R.A. Wilson, C.L. Hussey, Inorg. Chem. 1982, 21, 1263-1264; A.A.K. Abdul-Sada, P.W. Ambler, P.K.G. Hodgson, K.R. Seddon, N.J. Steward, WO-A-95/21871) R.H. Dubois, M.J. Zaworotko, P.S. White, Inorg. Chem. 1989, 28, 2019-2020; J.F. Knifton, J. Mol. Catal. 1987, 43, 65-78; C.P.M. Lacroix, F.H.M. Dekker, A.G. Talma, J.W.F. Seetz, EP-A-0989134). Ausgehend vom gebildeten und isolierten Halogenidsalz [A]⁺X⁻ sind zwei unterschiedliche Wege zur Synthese von ionischen Flüssigkeiten mit Hexafluorophosphat-, Tetrafluoroborat-, Bis(trifluormethytsulfonyl)-amid-, Perfluoralkylsulfonat- und Perfiluoralkylcarboxylat-Ionen bekannt. Zum einen wird das Halogenidsalz durch Zugabe eines Metallsalzes MY (unter Ausfällung oder Abscheidung des Salzes MX oder des Produktes [A]⁺[Y]⁻ aus dem jeweils verwendeten Lösungsmittel) umgesetzt - wobei [Y]⁻ ein Hexafluorophosphat-, Tetrafluoroborat-, Bis(trifluormethylsulfonyl)amid-, Perfluoralkylsulfonat- und

Perfluoralkylcarboxylat-Ion darstellt und M⁺ für ein Alkalikation steht (J.S. Wilkes, M.J. Zaworotko, J. Chem. Soc. Chem. Commun. 1992, 965-967; Y. Chauvin, L. Mußmann, H. Olivier, Angew. Chem. 1995, 107, 2941-2943; P.A.Z. Suarez, J.E.L. Dullius, S. Einloft, R.F. de Souza, J. Dupont, Polyhedron, 1996, 15, 1217-1219; P. Bonhöte, A.-P. Dias, N. Papageorgiou, K. Kalyanasundaram, M. Grätzel, Inorg. Chem. 1996, 35, 1168-1178; C.M.

Gordon, J.D. Holbrey, A.R. Kennedy, K.R. Seddon, J. Mater. Chem. 1998, 8, 2627-2638; P.A.Z. Suarez, S. Einloft, J.E.L. Dullius, R.F. de Souza, J. Dupont, J. Chim. Phys. 1998, 95, 1626-1639; A.J. Carmichael, C. Hardacre, J.D. Holbrey, M. Nieuwenhuyzen, K.R. Seddon, Anal. Chem. 1999, 71, 4572-4574; J.D. Holbrey, K.R. Seddon, J. Chem. Soc., Dalton Trans. 1999, 2133-2140). Zum anderen wird durch Zugabe einer starken Säure H⁺ [Y]⁻ das Halogenidion unter Freisetzung von H⁺ X⁻ verdrängt und gegen [Y]⁻ ausgetauscht - wobei [Y]⁻ hier für ein Hexafluorophosphat-, Tetrafluoroborat-, Bis(trifluormethylsulfonyl)amid-, Perfluoralkylsulfonat- und

Perfluoralkylcarboxylat-Ion steht (J. Fuller, R.T. Carlin, H.C. de Long, D. Haworth, J. Chem. Soc. Chem. Commun. 1994, 299-300). Besonders vorteilhaft jedoch können ionische Flüssigkeiten nach dem in der EP-A-1182196 beschriebenen Verfahren halogenidfrei hergestellt werden.

In einer Ausgestaltung des erfindungsgemäßen Verfahrens wird die ionische Flüssigkeit als alleiniges Reaktionsmedium, also frei von weiteren Lösungsmitteln eingesetzt. Der Anteil der ionischen Flüssigkeit im Reaktionsmedium kann aber auch zwischen 0,1 bis 99,9 Volumenprozent betragen, vorzugsweise zwischen 5 und 75 Volumenprozent, noch bevorzugter zwischen 15 oder 50 und 75 Volumenprozent, bezogen auf die Gesamtmenge des Reaktionsmediums.

Das Reaktionsmedium kann zusätzlich zu der ionischen Flüssigkeit noch ein weiteres Lösungsmittel enthalten. Dieses kann ausgewählt sein aus der Gruppe bestehend aus Wasser, Puffer-Lösungen (pH 2 bis 10, vorzugsweise 5 bis 8) und organischen Lösungsmitteln. Einsetzbare organische Lösungsmittel sind mit Wasser mischbar oder nicht mit Wasser mischbar. Beispielhaft seien als organische Lösungsmittel Methyl-tert.-butylether, Toluol, Hexan, Heptan, tert.-Butanol, Glycole, Polyalkylenglycole genannt. Im übrigen kommen aber grundsätzlich alle herkömmlichen, auf dem Gebiet der Enzymkatalyse bekannten Lösungsmittel in Frage.

Als Enzyme kommen grundsätzlich alle Enzyme der EC-Klassen 1 bis 6 in Frage. Die Klassifizierung von Enzymen wird vom "Nomenclature Committee of the International Union of Biochemistry and Molecular Biology" (IUBMB) empfohlen. Das Enzym liegt entweder homogen gelöst vor, kann aber ebenso als Suspension oder als Immobilisat auf einem inerten Träger eingesetzt werden.

Erfindungsgemäß wurde gefunden, dass die Gegenwart ionischer Flüssigkeiten im Reaktionsmedium bei enzymatisch katalysierten Reaktionen zu einer Verbesserung der Substratlöslichkeit (Biokompatibilität), zu einer Verbesserung der Enzymaktivität, zu einer Verbesserung der Selektivität, zu einer Verringerung von Produktinhibierung, zur Unterdrückung von Nebenreaktionen (Parallel-, Folgereaktionen) und/oder zu einer Erhöhung der Enzymstabilität führt. Die Beispiele belegen, dass Enzyme aus unterschiedlichen Klassen eingesetzt werden können, wobei der Einsatz ionischer Flüssigkeiten signifikante Vorteile bietet, wie beispielsweise eine Erhöhung der Aktivität bei der Formiat-Dehydrogenase, eine deutliche Erhöhung der Ausbeute bei Reaktionen mit der Galactosidase, eine Erhöhung der Enantioselektivität bei Lipasen und eine Verbesserung der Eduktlöslichkeit bei hydrophoben Edukten.

Erfindungsgemäß kann das Enzym zusammen mit der Gesamtmenge der ionischen Flüssigkeit oder einem Teil davon mehrfach oder in kontinuierlich betriebenen Reaktoren eingesetzt werden.

Die Enzyme können ausgewählt sein aus der Klasse der Oxidoreduktasen zur regio- und stereoselektiven Oxidation und Reduktion, aus der. Klasse der Glycosidasen zur Synthese von Oligosacchariden, aus der Klasse der Lipasen zur Gewinnung optisch aktiver Produkte (u. a. Alkohole, Amine, Carbonsäuren) aus der Klasse der Lyasen zur Synthese und Hydrolasen.

Das erfindungsgemäße Verfahren kann bei Temperaturen von -10 °C bis 130 °C, vorzugsweise in einem Temperaturbereich von 10 °C bis 80 °C, besonders bevorzugt in einem Temperaturbereich von 20°C bis 40°C durchgeführt werden.

Das Verfahren kann in einphasiger Weise oder in einem mehrphasigen Reaktionssystem durchgeführt werden.

Im Nachfolgenden sollen einige Effekte, die durch die Verwendung von ionischen Flüssigkeiten als Reaktionsmedium oder als Bestandteil von Reaktionsmedien für enzymatische Reaktionen beispielhaft erläutert werden. So werden für die enzymatische Reduktion von Ketonen unter anderem Alkoholdehydrogenasen aus verschiedenen Quellen eingesetzt. Die Löslichkeit von hydrophoben Ketonen kann durch Zusatz organischer Lösungsmittel verbessert werden; dies führt jedoch in der Regel zu einer Verringerung der Enzymaktivität und Stabilität (W. Hummel, Biochem. Eng. Biotechnol. 1997, 58, 145; A. Liese, T. Zelinski, M.-R. Kula, H. Kierkels, M. Karutz, U. Kragl, C. Wandrey, J. Mol. Cat. B 1998, 4, 91). In analoger Weise können wassermischbare ionische Flüssigkeiten zur Erhöhung der Eduktlöslichkeit dem Reaktionsmedium hinzugefügt werden. Für die Formiatdehydrogenase, die zur Cofaktorregenerierung eingesetzt wird, wird im gleichen Konzentrationsbereich der ionischen Flüssigkeit eine Erhöhung der Reaktionsgeschwindigkeit im Vergleich zum rein wässrigen System beobachtet (vgl. Beispiel 1) Eine Desaktivierung des Enzyms auch bei längerer Einwirkzeit der ionischen Flüssigkeit wurde nicht beobachtet. Somit bieten ionische Flüssigkeiten eine wertvolle Möglichkeit, die Produktivität enzymatischer Reaktionen durch einen Anstieg der Eduktkonzentration zu erhöhen. Dies ist besonders interessant für schlecht bis sehr schlecht lösliche Edukte wie etwa aromatische Ketone oder Steroide.

Seit etwa 20 Jahren werden Glycosidasen nicht nur für die Spaltung von Bindungen zwischen Sacchariden benutzt, sondern auch für die Synthese von Di- und Oligosacchariden. Trotz vieler Versuche, durch in der Regel teure Aktivierung der Edukte, Einsatz von wassermischbaren Lösungsmitteln (führt zu verringerter Enzymstabilität) sind selbst in neuesten Arbeiten Ausbeuten bis zu maximal 31% erreicht worden (J. H. Yoon, J. S. Rhee, Carbohydr. Res. 2000, 327, 377; M. J. Hernaiz, D. H. G. Crout, J. Mol. Cat. B 2000, 10, 403) Bei diesen Reaktionen ist das Hauptproblem die sofort einsetzende Sekundärhydrolyse des Produktes, katalysiert durch das gleiche Enzym. Überraschenderweise wird diese Sekundärhydrolyse in Gegenwart ionischer Flüssigkeiten bei sonst gleicher Aktivität des Enzyms fast vollständig unterdrückt. Für das Beispiel der β-Galactosidase-katalysierten Synthese von N-Acetyllactosamin, einem wichtigen Baustein für pharmakologisch relevante Oligosaccharide, konnte gezeigt werden, dass die Gegenwart ionischer Flüssigkeiten die Ausbeute bei Verwendung von Lactose als preiswertem Donor auf über 55% steigert! Ohne Zusatz ionischer Flüssigkeiten werden maximal 30% erreicht; allerdings nimmt die Produktkonzentration durch die Sekundärhydrolyse rasch auf Werte <10% ab. Da in Gegenwart ionischer Flüssigkeiten die Sekundärhydrolyse des Produktes unterbleibt, ergibt sich eine vereinfachte Reaktionsführung, da es nicht notwendig ist, die Reaktion zu verfolgen und beim Maximum der Produktausbeute abzubrechen. Die Galactosidase ist in Gegenwart ionischer Flüssigkeiten sehr stabil und kann nach Abtrennung mittels Ultrafiltration wiederholt eingesetzt werden, ohne dass sich die erzielbare Ausbeute und die Produktbildungsgeschwindigkeit verändert.

Vorteile bieten ionische Flüssigkeiten auch bei der Revershydrolyse zur Synthese von Di- und Oligosacchariden. Hierbei werden in der Regel hohe Eduktkonzentrationen zusammen mit Zusätzen - meist organischen Lösungsmitteln - zur Verringerung der Wasseraktivität verwendet. Ionische Flüssigkeiten bieten hier insbesonders den Vorteil des sehr guten Lösungsvermögen für Kohlenhydrate. Bei der enzymatischen Synthese von Lactose konnte gegenüber der Literatur die Ausbeute um den Faktor 2 gesteigert werden sowie die Reaktionszeit um den Faktor 5 gesenkt werden (K. Ajisaka, H. Fujimoto, H. Nishida, Carbohydr. Res. 180, 35-42 (1988))

Der Einsatz von Lipasen in Gegenwart von organischen Lösungsmitteln in ein-oder zweiphasiger Reaktionsführung ist Stand der Technik (G. Carrea, S. Riva, Angew. Chem. 2000, 112, 2312, U. T. Bornscheuer, R. J. Kazlauskas, Hydrolases in Organic Synthesis - Regio- and stereoselective biotransformations, Wiley-VCH, Weinheim, 1999; A. Liese, K. Seelbach, C. Wandrey, Industrial Biotransfomrations, Wiley-VCH, Weinheim, 2000; M. C. Parker, S. A. Brown, L. Robertson, N. J. Turner, Chem. Commun. 1998, 2247). Allerdings wurde bisher in konventioneller Weise das Enzym durch Filtration abgetrennt und die Reaktionslösung konventionell durch Destillation aufgetrennt und das Lösungsmittel zurückgeführt. Der Einsatz ionischer Flüssigkeiten gestattet die direkte destillative Abtrennung der Reaktanden aus der Reaktionsmischung selbst in Gegenwart des Enzyms, so dass sich eine vereinfachte Verfahrensweise ergibt. Diese Verfahrensweise ist, wenn die Reaktanden entsprechende Flüchtigkeit besitzen, nicht auf Lipasen beschränkt. Bei der Untersuchung unterschiedlicher Lipasen für die Racematspaltung in Gegenwart ionischer Flüssigkeiten wurde überraschenderweise in mehreren Fällen festgestellt, dass sich die Umsatzgeschwindigkeit und die Enantioselektivität zum Teil deutlich verbessert, im Einzelfall um den Faktor 5 besser. Als Vergleich dient die Reaktion in *tert*.-Butylmethylether, der auch in industriellen Prozessen als Lösungsmittel für Lipase-katalysierte Reaktionen eingesetzt wird.

Die Ergebnisse zeigen, dass ionische Flüssigkeiten als Reaktionsmedium für enzymatische Umsetzungen zahlreiche Vorteile gegenüber den Bedingungen, die als Stand der Technik etabliert sind, bieten und als biokompatible Lösungsmittel genutzt werden können, um Umsetzungen gezielt zu beeinflussen.

Die Erfindung wird durch die nachfolgenden Beispiele näher beschrieben ohne jedoch auf diese beschränkt zu sein.

### Beispiele:

Zur Beschreibung von in den Beispielen verwendeten Komponenten wurden die folgenden Abkürzungen verwendet:

| | | |
|---|---|---|
| *tert*.-Butylmethylether | tBME, | MTBE |
| Butyl-Methyl-Imidazolium PF₆⁻ | BMIm⁺ | PF₆⁻ |
| Nonyl-Methyl-Imidazolium PF₆⁻ | NMIm⁺ | PF₆⁻ |
| Butyl-Methyl-Imdazolium BF₄⁻ | BMIm⁺ | BF₄⁻ |
| Hexyl-Methyl-Imdazolium BF₄⁻ | HMIm⁺ | BF₄⁻ |
| Octyl-Methyl-Imdazolium BF₄⁻ | OMIm⁺ | BF₄⁻ |
| 4-Methyl-Butyl-Pyridinium BF₄⁻ | 4-MBPy⁺ | BF₄⁻ |
| Triethylammonium-Methylsulfat | Et₃NH⁺ | MeSO₄⁻ |
| Triethylmethylammonium-Methylsulfat | Et₃NMe⁺ | MeSO₄⁻ |
| Butylmethyl-Pyridinium BF₄⁻ | BMPy⁺ | |
| BF₄⁻ | | |
| Propylammonium-Nitrat | PrNH₃⁺ | NO₃⁻ |
| Methyl-Methyl-Imidazolium-Methylsulfat | MMIm⁺ | MeSO₄⁻ |
| Ethyl-Methyl-Imidazolium-Benzoat | EMIm⁺ | PhCO₂⁻ |
| Butyl-Methyl-Imidazolium-Trifluormethansulfonat | BMIm⁺ | CF₃SO₃⁻ |
| | | (= Triflat) |
| Butyl-Methyl-Imidazolium-Bis-(Trifluormethyl sulfonyl)-imidat | BMIm⁺ (CF₃SO₂)₂N⁻ | |

### 1. Formiatdehydrogenase aus Candida boidinii (FDH)

Als Testreaktion zur Bestimmung der Enzymaktivität dient die FDHkatalysierte Oxidation von Ameisensäure zu Kohlenstoffdioxid unter Reduktion von Nicotinamidadenindinucleotid (NAD⁺ zu NADH + H⁺). Im Enzymassay wird die zeitliche NADH-Zunahme bei 25 °C photometrisch bei einer Wellenlänge von 340 nm detektiert.

Zusammensetzung des Enzymassay: 1 ml Puffer-Lösung (50 mM Triethanolamin- Hydrochlorid, 1 mM Dithiothreitol, Salzsäure) pH 7 wird mit 0,1 ml wässriger Natriumformiat-Lösung (2,4 M ) und 0,1 ml Enzym-Lösung (0,7 mg/ml, 8,4 U) versetzt. Die Enzym-Lösung enthält bereits den Cofaktor NAD (6 mM).

Um den Einfluss wasserlöslicher ionischer Flüssigkeiten auf die Enzymaktivität zu testen, wird das Volumen der Puffer-Lösung im Assay schrittweise um 25 vol% reduziert und durch die ionische Flüssigkeit ersetzt.

**Tabelle 1 NAD-Reduktion durch Formiatdehydrogenase aus Candida boidinii; Enzymaktivität im Vergleich zur Standardreaktion in Pufferlösung (+) gut bis besser, (±) gleich.**

| **Ionische Flüssigkeit** | **25 vol%** | **50 vol%** | **75 vol%** |
|---|---|---|---|
| MMIm⁺ MeSO₄⁻ | ± | + | + |

### 2. β-Galactosidase aus Bacillus circulans zur Synthese von N-Acetyllactosamin

Es wird der Einfluss ionischer Flüssigkeiten auf den Verlauf der β-Galkatalysierten Transgalactosylierung ausgehend von Lactose und *N-*Acetylglucosamin untersucht. Hierzu werden Konzentrations-Zeit-Verläufe dieser Synthese in Gegenwart und Abwesenheit ionischer Flüssigkeiten aufgenommen und verglichen.

Je Versuchsreihe werden in 1 ml GC-Gläschen 10 Reaktionen parallel angesetzt. Im 10-Minuten-Intervall werden die Reaktionen durch 10 minütiges Kochen bei 100 °C gestoppt, die Reaktionslösung filtriert (Spritzenfilter Minisart RC 4 Sartorius) und die Konzentration der Reaktionskomponenten zu diesem Zeitpunkt chromatographisch bestimmt (Kationentauschersäule Aminex HPX-87H der Firma BioRad mit entsprechender Vorsäule, 0,006 M Schwefelsäure als Eluent mit einem Fluss von 0,8 ml/min und einer Säulentemperatur von 65 °C. Die Detektion erfolgt mittels UV bei 208 nm und mittels Brechungsindex).

Zusammensetzung der Reaktionsmischung: 0,05 ml Puffer-Lösung (65 mM KH₂PO₄, 195 mM K₂HPO₄) pH 7,3 werden mit 0,5 ml N-Acetylglucosamin-Lösung (GlcNAc 600 mM bzw. 1,2 M in Puffer-Lösung), 0,25 ml Lactose-Lösung (250 mM in Puffer-Lösung) und 0,2 ml Enzym-Lösung (10 mg/mlin Puffer-Lösung) versetzt.

Durch Austausch von Puffer-Lösung gegen ionische Flüssigkeit in den Substrat-Lösungen wird der Anteil an ionischer Flüssigkeit im Reaktionsmedium schrittweise erhöht. Damit ergeben sich folgende Substratlösungen:
a) 0,50 ml *N*-Acetylglucosamin-Lösung (600 mM in 1:4 MMIm⁺ MeSO₄⁻ : Puffer-Lösung)
b) 0,50 ml *N*-Acetylglucosamin-Lösung (600 mM in 1:4 MMIm⁺ MeSO₄⁻ : Puffer-Lösung)
   0,25 mL Lactose-Lösung (250 mM in 1:4 MMIm⁺ MeSO₄⁻ : Puffer-Lösung)
c) 0,50 ml *N*-Acetylglucosamin-Lösung (1,2 M in 1:2 MMIm⁺ MeSO₄⁻ : Puffer-Lösung)
   0,25 mL Lactose-Lösung (250 mM in 1:2 MMIm⁺ MeSO₄⁻ : Puffer-Lösung)
d) 0,50 ml N-Acetylglucosamin-Lösung (600 mM in 1:4 BMIm⁺ H₂PO₄⁻/Cl⁻ : Puffer-Lsg.)

**Tabelle 2 Synthese von N-Acetyllactosamin durch β-Galactosidase aus Bacillus circulans mit und ohne ionische Flüssigkeit**

| **Reaktions-medium** | **Anteil [vol%]** | **Lactose/ GIcNAc Verhältnis** | **Ausbeute [%] nach 60 min** | **Ausbeute [%] nach 100 min** |
|---|---|---|---|---|
| Phosphat-Puffer | | 1 : 2,4 | 5 | 3 |
| MMIm⁺ MeSO₄⁻ | a) 12,5 | 1 : 2,4 | 40 | 40 |
| | b) 18,75 | 1 : 2,4 | 44 | 43 |
| | c) 25 | 1 : 4,8 | 49 | 55 (90 min) |
| BMIm⁺ H₂PO₄⁻/Cl⁻ | d) 12,5 | 1 : 2,4 | 39 | 30 |

### 3. Enantioselektive Acylierung von R,S-1-Phenylethanol durch Katalyse mit Lipase aus Candida antarctica (Type B) in ionischen Flüssigkeiten

4,4 ml einer ionischen Flüssigkeit entsprechend Tabelle 3 oder *tert*.-Butylmethylether werden mit 122µl Vinylacetat und 54 µl 1-Phenylethanol versetzt, sodass eine Substratlösung mit etwa 0,1 mol/l 1-Phenylethanol und 0,3 mol/l Vinylacetat erhalten wird. Je 1 mg lyophilisierte Lipase (>120 U/mg) wird mit 0,4 ml Substratlösung versetzt, gründlich gemischt und im Thermoschüttler bei 24 °C unter leichtem Schütteln 3-4 d inkubiert.
Zur Aufarbeitung werden 100 µl des Reaktionsansatzes mit 1 ml n-Hexan/Isopropanol (97,5/2,5 v/v) versetzt und gründlich gemischt. Dieser Hexan/Isopropanol-Extrakt wird zur Bestimmung der Konzentrationen und Enantiomerenverhältnisse von 1-Phenylethanol und 1-Phenylethylacetat mittels HPLC eingesetzt. Aus diesen Konzentrationen wurden der Umsatz und der Enantiomerenüberschuss berechnet (siehe Tabelle 3).

### HPLC-Bedingungen:

- Säule:: Schutzsäule Nucleosil C-18 5 µm; 10 mm, 4,6 mm ID; Vorsäule Chiracel OJ; 50 mm, 4,6 mm ID; Trennsäule Chiracel OJ; 250 mm, 4,6 mm ID
- Eluent:: isokratisch;
96,5 % (v/v) n-Hexan,
3,0 % (v/v) Isopropanol,
0,5 % (v/v) Ethanol
- Flussrate:: 1 ml/min
- Temperatur:: 38 °C
- Detektion:: UV-Detektor (205 nm)

**Tabelle 3 Enantioselektive Acylierung von 1-Phenylethanol, katalysiert durch Lipase aus Candida antarctica (Type B): Umsatz und Enantiomerenüberschuss in ionischen Flüssigkeiten im Vergleich zur Standardreaktion in tert.-Butylmethylether; (+) gut bis besser, (±) gleich, (-) schlecht bis kein.**

| **Ionische Flüssigkeit/ Lösungsmittel** | **Umsatz** | **Enantiomerenüberschuss** |
|---|---|---|
| NMIm⁺ PF₆⁻ | ± | + |
| BMIm⁺ BF₄⁻ | + | + |
| HMIm⁺ BF₄⁻ | ± | + |
| OMIm⁺ BF₄ | + | + |
| 4-MBP⁺ BF₄ | + | + |
| BMIm⁺ CF₃SO₃⁻ | + | + |
| BMIm⁺ (CF₃SO₂)₂N⁻ | + | + |

### 4. Enantioselektive Acylierung von R,S-1-Phenylethanol durch Katalyse mit Lipase aus Candida antarctica (Type A) in ionischen Flüssigkeiten

Je 5 mg lyophilisierte Lipase (>30 U/mg) werden mit 0,4 ml einer Substratlösung wie in Beispiel 3 vermischt. Die weitere Arbeitsweise entspricht der in Beispiel 3 beschriebenen.

**Tabelle 4 Enantioselektive Acylierung von 1-Phenylethanol, katalysiert durch Lipase aus Candida antarctica (Type A): Umsatz und Enantiomerenüberschuss in ionischen Flüssigkeiten im Vergleich zur Standardreaktion in tert.-Butylmethylether; (+) gut bis besser, (±) gleich, (-) schlecht bis kein.**

| **Ionische Flüssigkeit/ Lösungsmittel** | **Umsatz** | **Enantiomerenüberschuss** |
|---|---|---|
| BMIm⁺ PF₆⁻ | + | + |
| NMlm⁺ PF₆⁻ | + | + |
| BMIm⁺ BF₄⁻ | ± | + |
| HMIm⁺ BF₄⁻ | + | + |
| OMIm⁺ BF₄⁻ | + | ± |
| BMIm⁺ CF₃SO₃⁻ | + | + |

### 5. Enantioselektive Acylierung von R,S-1-Phenylethanol durch Katalyse mit Lipase aus Pseudomonas sp. in ionischen Flüssigkeiten

Je 3 mg lyophilisierte Lipase (400 U/mg) werden mit 0,4 ml einer Substratlösung wie in Beispiel 3 vermischt. Die weitere Arbeitsweise entspricht der in Beispiel 3 beschriebenen.

**Tabelle 5 Enantioselektive Acylierung von 1-Phenylethanol, katalysiert durch Lipase aus Pseudomonas sp. : Umsatz und Enantiomerenüberschuss in ionischen Flüssigkeiten im Vergleich zur Standardreaktion in tert.-Butylmethylether; (+) gut bis besser, (±) gleich, (-) schlecht bis kein.**

| **Ionische Flüssigkeit/ Lösungsmittel** | **Umsatz** | **Enantiomerenüberschuss** |
|---|---|---|
| MIm⁺ PF₆⁻ | ± | + |
| 4-MBP⁺ BF₄⁻ | ± | + |
| BMIm⁺ CF₃SO₃⁻ | + | + |
| BMIm⁺ (CF₃SO₂)₂N⁻ | + | + |

### 6. Enantioselektive Acylierung von R,S-1-Phenylethanol durch Katalyse mit Lipase aus Alcaligines sp. in ionischen Flüssigkeiten

Je 5 mg lyophilisierte Lipase (>20 U/mg) werden mit 0,4 ml einer Substratlösung wie in Beispiel 3 vermischt. Die weitere Arbeitsweise entspricht der in Beispiel 3 beschriebenen.

**Tabelle 6 Enantioselektive Acylierung von 1-Phenylethanol, katalysiert durch Lipase aus Alcaligines sp.: Umsatz und Enantiomerenüberschuss in ionischen Flüssigkeiten im Vergleich zur Standardreaktion in tert.-Butylmethylether; (+) gut bis besser, (±) gleich, (-) schlecht bis kein.**

| **Ionische Flüssigkeit/ Lösungsmittel** | **Umsatz** | **Enantiomerenüberschuss** |
|---|---|---|
| BMIm⁺ PF₆⁻ | ± | + |
| BMIm⁺ BF₄⁻ | ± | + |
| HMIm⁺ BF₄⁻ | ± | + |
| OMIm⁺ BF₄⁻ | ± | + |
| 4-MBP⁺ BF₄⁻ | ± | + |
| BMIm⁺ CF₃SO₃⁻ | ± | + |

### 7. Recyclierung der Lipase aus Candida antarctica (Type B) in ionischer Flüssigkeit durch Destillation

600 mg lyophilisierte Lipase (ca. 10 U/mg) werden mit 4 ml ionischer Flüssigkeit (BMIm⁺ (CF₃SO₂)₂N⁻), 1,2 ml Vinylacetat und 0,7 ml 1-Phenylethanol versetzt und gründlich gemischt. Die Reaktionsmischung wird 40 min bei 40 °C inkubiert.
Anschließend werden die nicht umgesetzten Edukte sowie das Reaktionsprodukt 1-Phenylacetat abdestilliert (85 °C, 0,06 mbar).
Die Enzym/ionische Flüssigkeit-Mischung wird abgekühlt, erneut mit 1,2 ml Vinylacetat und 0,7 ml 1-Phenylethanol versetzt und wiederum 40 min bei 40 °C inkubiert.

Diese Reaktionsfolge von Inkubation und Abdestillieren kann mehrfach wiederholt werden, ohne dass die Aktivität der Lipase zurückgeht.

### 8. Synthese von Lactose durch Revershydrolyse mit β-Galactosidase aus Bacillus circulans

100 mmol/l Glucose, 20 mmol/l Galactose und 2 mg/ml Glactosidase werden bei 35 °C im Gemisch aus Wasser und MMIm MeSO₄ für 24 h inkubiert. Die Reaktion wird durch 10 minütiges Kochen bei 100 °C gestoppt, die Reaktionslösung filtriert (Spritzenfilter Minisart RC 4 Sartorius) und die Konzentration der Reaktionskomponenten zu diesem Zeitpunkt chromatographisch bestimmt (Kationentauschersäule Aminex HPX-87H der Firma BioRad mit entsprechender Vorsäule, 0,006 M Schwefelsäure als Eluent mit einem Fluss von 0,8 ml/min und einer Säulentemperatur von 65 °C. Die Detektion erfolgt mittels UV bei 208 nm und mittels Brechungsindex). Der Anteil der ionischen Flüssigkeit wird von 0 bis 100 Volumenprozent erhöht. Durch Wasserspuren in der ionischen Flüssigkeit und den Edukten ergibt sich ein Wassergehalt bei 100% ionischer Flüssigkeit von 0,6%. Nach 24 Stunden steigt der Umsatz nicht mehr weiter an. In Abhängigkeit von der Menge an ionsicher Flüssigkeit werden folgende Lactoseausbeuten erhalten:

| **Anteil ionischer Flüssigkeit in %** | **Ausbeute [%]** |
|---|---|
| 0 | 0 |
| 10 | 0 |
| 20 | 0 |
| 30 | 4 |
| 40 | 12 |
| 50 | 15 |
| 60 | 15 |
| 70 | 16 |
| 80 | 16 |
| 90 | 16 |
| 100 | 17 |

## Patentansprüche

1. Verfahren zur Umsetzung von Substanzen in Gegenwart von Enzymen als Katalysator in einem Reaktionsmedium umfassend wenigstens eine ionische Flüssigkeit, wobei die Enzyme ausgewählt sind aus der Gruppe der Oxidoreductasen, Lipasen, Galactosidasen, Glycosidasen, Lyasen, und Enzymen der EC-Klasse 6.

2. Verfahren nach Anspruch 1, **dadurch gekennzeichnet, dass** der Anteil der ionischen Flüssigkeit im Reaktionsmedium 0,1 bis 99,9 Volumenprozent beträgt.

3. Verfahren nach einem der vorangehenden Ansprüche, **dadurch gekennzeichnet, dass** das Reaktionsmedium zusätzlich zur der ionischen Flüssigkeit noch ein weiteres Lösungsmittel erhält.

4. Verfahren nach einem der vorangehenden Ansprüche, **dadurch gekennzeichnet, dass** das weitere Lösungsmittel Wasser oder ein organisches Lösungsmittel ist.

5. Verfahren nach einem oder mehreren der vorangehenden Ansprüche, **dadurch gekennzeichnet, dass** die Reaktion bei Temperaturen von -10 °C bis 130 °C durchgeführt wird.

6. Verfahren nach einem der vorangehenden Ansprüche, **dadurch gekennzeichnet, dass** die Reaktion in einphasiger Weise oder in einem mehrphasigen Reaktionssystem durchgeführt wird.

7. Zusammensetzung umfassend ein Enzym, ausgewählt aus der Gruppe der Oxidoreductasen, Lipasen, Galactosidasen, Glycosidasen, Lyasen, und Enzymen der EC-Klasse 6 und wenigstens eine ionische Flüssigkeit.

8. Zusammensetzung nach Anspruch 7, **dadurch gekennzeichnet, dass** die ionische Flüssigkeit wie in Anspruch 2 definiert ist.

9. Zusammensetzung nach Anspruch 7 oder 8, **dadurch gekennzeichnet, dass** sie zusätzlich ein Substrat enthält.

10. Zusammensetzung nach irgendeinem der Ansprüche 7 bis 9, **dadurch gekennzeichnet, dass** sie die ionische Flüssigkeit als Reaktionsmedium oder als Bestandteil des Reaktionsmediums enthält.

11. Verwendung von ionischen Flüssigkeiten als Reaktionsmedium oder als Bestandteil des Reaktionsmediums für enzymatisch katalysierte Reaktionen, wobei die Enzyme ausgewählt sind aus der Gruppe der Oxidoreductasen, Lipasen, Galactosidasen, Glycosidasen, Lyasen, und Enzymen der EC-Klasse 6.

12. Verwendung gemäß Anspruch 11 als Reaktionsmedium oder als Bestandteil des Reaktionsmediums bei der Revershydrolyse zur Synthese von Di- und Oligosacchariden.

## Claims

1. A process for reacting substances in the presence of enzymes as catalysts in a reaction medium comprising at least one ionic liquid, wherein said enzymes are selected from the group consisting of oxidoreductases, lipases, galactosidases, glycosidases, lyases and enzymes of E.C. class 6.

2. The process according to claim 1, **characterized in that** the proportion of ionic liquid in the reaction medium is from 0.1 to 99.9% by volume.

3. The process according to any of the preceding claims, **characterized in that** the reaction medium contains a further solvent in addition to said ionic liquid.

4. The process according to any of the preceding claims, **characterized in that** said further solvent is water or an organic solvent.

5. The process according to one or more of the preceding claims, **characterized in that** the reaction is performed at temperatures of from -10 °C to 130 °C.

6. The process according to any of the preceding claims, **characterized in that** the reaction is performed in one-phase mode or in a multiphase reaction system.

7. A composition comprising an enzyme selected from the group consisting of oxidoreductases, lipases, galactosidases, glycosidases, lyases and enzymes of E.C. class 6, and at least one ionic liquid.

8. The composition according to claim 7, **characterized in that** said ionic liquid is defined as in claim 2.

9. The composition according to claim 7 or 8, **characterized by** additionally containing a substrate.

10. The composition according to any of claims 7 to 9, **characterized by** containing said ionic liquid as a reaction medium or as a component of the reaction medium.

11. Use of ionic liquids as a reaction medium or as a component of the reaction medium for enzymatically catalyzed reactions, wherein said enzymes are selected from the group consisting of oxidoreductases, lipases, galactosidases, glycosidases, lyases and enzymes of E.C. class 6.

12. The use according to claim 11 as a reaction medium or as a component of the reaction medium in the reverse hydrolysis for the synthesis of disaccharides and oligosaccharides.

## Revendications

1. Procédé pour la réaction de substances en présence d'enzymes en tant que catalyseur dans un milieu réactionnel comprenant au moins un liquide ionique, les enzymes étant choisies dans le groupe des oxydoréductases, lipases, galactosidases, glycosidases, lyases et enzymes de la classe EC 6.

2. Procédé selon la revendication 1, **caractérisé en ce que** la proportion de liquide ionique dans le milieu réactionnel est de 0,1 à 99,9 % en volume.

3. Procédé selon l'une des revendications précédentes, **caractérisé en ce que** le mélange réactionnel reçoit en plus du liquide ionique encore un autre solvant.

4. Procédé selon l'une des revendications précédentes, **caractérisé en ce que** l'autre solvant est de l'eau ou un solvant organique.

5. Procédé selon une ou plusieurs des revendications précédentes, **caractérisé en ce que** la réaction est conduite à des températures de -10°C jusqu'à 130°C.

6. Procédé selon l'une des revendications précédentes, **caractérisé en ce que** la réaction est conduite en une seule phase ou dans un système réactionnel polyphasé.

7. Composition comprenant une enzyme, choisie dans le groupe des oxydoréductases, lipases, galactosidases, glycosidases, lyases, et enzymes de la classe EC 6 et au moins un liquide ionique.

8. Composition selon la revendication 7, **caractérisée en ce que** le liquide ionique est tel que défini dans la revendication 2.

9. Composition selon la revendication 7 ou 8, **caractérisée en ce qu'**elle contient en outre un substrat.

10. Composition selon l'une quelconque des revendications 7 à 9, **caractérisée en ce qu'**elle contient le liquide ionique en tant que milieu réactionnel ou comme composant du milieu réactionnel.

11. Utilisation de liquides ioniques en tant que milieu réactionnel ou comme composant du milieu réactionnel pour des réactions catalysées par voie enzymatique, les enzymes étant choisies dans le groupe des oxydoréductases, lipases, galactosidases, glycosidases, lyases, et enzymes de la classe EC 6.

12. Utilisation selon la revendication 11 en tant que milieu réactionnel ou comme composant du milieu réactionnel dans l'hydrolyse réversible pour la synthèse de di- ou oligosaccharides.
